# EUROPÄISCHE PATENTANMELDUNG

(11) **EP 2 229 885 A1**
(43) Veröffentlichungstag der Anmeldung: **22.09.2010**
(21) Anmeldenummer: 10153808.0
(22) Anmeldetag: 17.02.2010
(51) Int. Cl.: A61B 5/145, A61B 8/00, G01N 33/574

(54) **Verfahren zur Ermittlung einer Tumorkenngröße und Medizingerät**

(30) Priorität: 19.03.2009 DE 102009014052
(71) Anmelder: SIEMENS AKTIENGESELLSCHAFT, 80333 München (DE)
(72) Erfinder: Chaussy, Christian, 82064, Strasslach (DE); Thüroff, Stefan, 81545, München (DE); Bergsdorf, Thorsten, 82064, Straßlach (DE); Nanke, Ralf, 91077, Neunkirchen am Brand (DE)

(57) **Zusammenfassung**

Bei einem Verfahren zur Ermittlung einer auf eine Körperregion (12) eines Patienten (2) bezogenen Tumorkenngröße (K):
- wird die Körperregion (12) zum Zweck einer mechanischen Irritation mit akustischer Energie (8) bestrahlt,
- wird zu einem ersten Zeitpunkt (t1) ein erster Wert (18a) eines mit der Irritation korrelierten Kennwertes (B) des Patienten (2) ermittelt,
- wird zu einem nach dem ersten Zeitpunkt (t1) und nach der Bestrahlung liegenden zweiten Zeitpunkt (t2) ein zweiter Wert (18b) des Kennwertes (B) ermittelt,
- wird die Tumorkenngröße (K) aus der Änderung zwischen erstem (18a) und zweitem Wert (18b) ermittelt.

## Beschreibung

Die Erfindung betrifft ein Verfahren zur Ermittlung einer Tumorkenngröße und ein entsprechend hierzu geeignetes Medizingerät.

Eine Diagnose, welche in der Regel ein Arzt bezüglich eines Patienten stellt, ist meist auf eine Vielzahl von Kenngrößen in Form von Mess- bzw. Kennwerten gestützt. Diese Werte werden am Patienten ermittelt bzw. gewonnen. Beispiele sind medizinische Bilddaten wie Röntgenbilder, MR- oder CT-Aufnahmen oder auch Messwerte wie Pulsfrequenz, Blutdruck, Blutzusammensetzung bzw. -kennwerte. Kenngrößen, welche der Diagnose einer Tumorerkrankung am Patienten dienen, heißen Tumorkenngrößen. Kenngrößen, welche Kennwerte liefern, die auf eine bestimmte Körperregion eines Patienten, z.B. dessen Leber, Milz, Prostata, Hoden oder Brust begrenzt sind, heißen "auf diese Körperregion bezogene" Tumorkenngrößen.

Diese Kenngrößen leisten dem Arzt Hilfestellung bei seiner Diagnose, ob nämlich die betreffende Körperregion von einem Tumor befallen ist oder nicht. Anhand der Diagnose entscheidet der Arzt dann über eine geeignete, am Patienten durchzuführende Therapie. Derartige Tumorkenngrößen werden in entsprechenden am Patienten durchzuführenden Verfahren ermittelt. Diese Verfahren heißen daher auch diagnose- oder therapieunterstützende Methoden und werden zusammen mit der Diagnose als Diagnostik bezeichnet.

Eine exakte Diagnostik ist ein maßgeblicher Baustein in der Krebsvorsorge und -therapie an Patienten. Je früher und genauer eine Diagnose gestellt werden kann, umso effizienter kann die auf die Diagnose hin eingeleitete Therapie gestaltet werden.

Hinzu kommt, dass je billiger, sicherer und weniger invasiv eine derartige Methode ist, umso wahrscheinlicher ist ihre Anwendung in der medizinischen Praxis.

Insbesondere für Prostatakarzinome (PCa) ist eine bildgebende Diagnostik wenig effizient und teuer. Die Entscheidung, ob ein PCa bei einem Patienten vorhanden ist oder sogar schon Lymphknoten des Patienten metastasiert sind, ist jedoch ein wichtiges Kriterium für die anschließende Therapie.

Der bisherige Goldstandard zur Feststellung von Lymphknotenmetastasen ist eine Histologie. Neben dieser operativen Lymphknotendiagnostik ist es seit einiger Zeit auch bekannt, sehr teure und aufwendige Bildgebungsverfahren wie kontrastmittelverstärkte Magnetresonanztomographie (MRT) oder Positronen-Emissions-Tomographie/Computer-Tomographie (PET-CT) einzusetzen.

Ein wichtiger Baustein bei der PCa-Diagnose ist unter anderem die Bestimmung der Konzentration des Prostataspezifischen Antigens (PSA-Konzentration) als Tumorkenngröße. PSA ist ein im Serum messbarer Marker für Prostatagewebe, der nicht spezifisch für PCa ist. Prostatakarzinome sind in ca. 90 % aller Fälle mit einem erhöhten PSA-Wert verbunden.

Die Tumorkenngröße muss also eine möglichst differenzierte Aussage liefern, die der Arzt bei seiner Entscheidung darüber nutzt, ob eine bestimmte Körperregion eines Patienten tumorbehaftet ist oder nicht. Die Entscheidung, ob anhand eines gemessenen PSA-Wertes Krebsverdacht besteht oder nicht, ist für den Arzt schwierig, da sich die PSA-Werte gesunder und von Krebs befallener Patienten oftmals nur geringfügig unterscheiden können. Dies gilt auch für andere Serummarker, die als oder in Verbindung mit Tumorkenngrößen Verwendung finden, z.B. Alpha-Fetoprotein (AFP) oder Beta Humanes Chorion-Gonadotropin (bHCG). AFP und b-HCG sind hierbei nicht gewebespezifisch und lassen somit keinen zuverlässigen Rückschluss auf den Primärtumor zu; eine massive b-HCG bzw. β-HCG Erhöhung findet man z.B. auch bei Vorliegen einer Schwangerschaft.

Aufgabe der vorliegenden Erfindung ist es daher, ein verbessertes Verfahren zur Ermittlung einer Tumorkenngröße und ein hierfür geeignetes Medizingerät anzugeben.

Die Erfindung beruht auf der Erkenntnis, dass Prostatagewebe bei mechanischer Irritation, wie sie z.B. bei einer digital rektalen Untersuchung stattfindet, PSA in die Blutbahn abgibt. Durch den Druck auf das Gewebe erhöht sich der im Blutserum des Patienten gemessene PSA-Wert vorübergehend. Außerhalb der Prostatakapsel kann nur malignes Prostatagewebe gefunden werden. Auch hier sollte - wie bei den intraprostatischen Prostatazellen - durch eine mechanische Irritation ein PSA-Anstieg nachweisbar sein.

Die Erfindung beruht auf der Idee, die oben festgestellte Tatsache für eine verbesserte Ermittlung einer Tumorkenngröße auszunutzen. Grundlegende Idee ist, eine direkte und gezielte mechanische Irritation des zu betrachtenden Organs bzw. der betreffenden Körperregion des Patienten durchzuführen. Mit anderen Worten wird eine spezielle Körperregion eines Patienten mechanisch irritiert, die daraufhin einen Serummarker in höherer Konzentration als sonst freisetzt. Die Konzentration des Serummarkers im Blut wird also künstlich erhöht. Damit handelt es sich um ein Reizdiagnostikverfahren. Der künstlich induzierte Anstieg der Konzentration des Serummarkers im Blut des Patienten erlaubt es dem Arzt, aus der betreffenden Tumorkenngröße einen sichereren pathologischen Befund bzw. diagnostischen Schluss zu ziehen.

Die Erfindung beruht auf der Idee, die mechanische Irritation durch das Einbringen akustischer Energie in die betreffende Körperregion zu erzeugen, um so Zellen im irritierten Gebiet zur Ausschüttung von im Blutkreislauf nachweisbaren Faktoren, wie z.B. Tumormarkern, zu provozieren. Dies sollte bei sämtlichen Tumoren, z.B. Prostatakarzinom, Hodenkarzinom, Mammakarzinom usw. funktionieren, welche an einem Primärorgan Tumormarker exprimieren.

Die Aufgabe wird hinsichtlich des Verfahrens gelöst durch ein Verfahren gemäß Patentanspruch 1 zur Ermittlung einer Tumorkenngröße, welche sich auf eine Körperregion eines Patienten bezieht.

Bei dem Verfahren wird die Körperregion mit akustischer Energie bestrahlt. Die akustische Energie dient zum Zweck einer mechanischen Irritation der Körperregion. Mit anderen Worten wird durch Einbringen akustischer Energie die Körperregion mit einem mechanischen Reiz beaufschlagt. Zu einem ersten Zeitpunkt wird dann ein erster Wert eines Kennwertes des Patienten ermittelt. Der Kennwert ist hierbei mit der Irritation korreliert. Mit anderen Worten wird am Patienten ein Kennwert bestimmt, welcher sich bekannterweise durch die mechanische Irritation der Körperregion gegenüber dem ungereizten Zustand verstärkt, abschwächt oder in anderer Weise hervorhebt. Die Körperregion wird hierbei in einem gepulsten Betrieb oder auch kontinuierlich bestrahlt.

Zu einem zweiten Zeitpunkt wird dann ein zweiter Wert desselben Kennwertes ermittelt. Der zweite Zeitpunkt liegt hierbei nach dem ersten Zeitpunkt und auch nach dem Zeitpunkt der Bestrahlung der Körperregion mit akustischer Energie, also nach der Irritation. Die Tumorkenngröße wird dann aus der Änderung des Kennwertes zwischen erstem und zweitem Zeitpunkt, also aus der Änderung zwischen erstem und zweitem Wert ermittelt. Sinnvollerweise sollte der erste Wert als Basiswert vor der Irritation bestimmt werden. Jedoch sind auch andere Vorgehensweisen möglich, wie später erläutert wird.

Im erfindungsgemäßen Verfahren wird also eine gezielte Stimulation der Körperregion, also z.B. eines Organs oder von Lymphknoten, durchgeführt, um einen Kennwert für diagnostische Zwecke bezüglich seiner Aussagekraft als Tumorkenngröße zu verstärken, z.B. eine Serummarkerausschüttung zu erhöhen.

Der Kennwert ist vorzugsweise ein solcher, der aus einer Körperflüssigkeit des Patienten ermittelbar ist, z.B. ein Blut- oder Urinkennwert.

Das Verfahren bietet eine neuartige Form der Vorsorge bzw. Erstdiagnostik und einen Informationsgewinn im zeitlichen Verlauf des Kennwertes, z.B. dem Serummarkerverlauf, sowie der Höhe eines messbaren Ausschlages z.B. im zeitlichen Verlauf des Blutkennwertes. Das Verfahren bietet eine elegante, nichtinvasive, schnelle und billige Ermittlung der Tumorkenngröße, z.B. zur Lymphknotendiagnostik. Operationen zur Gewinnung histologischer Proben können hierdurch eingespart werden. Die durch die mechanische Irritation künstlich hervorgerufene verstärkte Änderung der Werte des Kennwertes ist nur vorübergehend, z.B. ist eine PSA-Erhöhung 24 Stunden nach der mechanischen Irritation nicht mehr nachweisbar. Das erfindungsgemäße Verfahren kann daher im Rahmen weiterer Diagnosen wiederholt werden, z.B. um eine Verlaufskontrolle am Patienten durchzuführen. Das Verfahren ist vom genannten PCa-/PSA-Beispiel auf viele andere medizinische Bereiche übertragbar. Das erfindungsgemäße Verfahren wird bei Verwendung von Stoßwellen zur Irritation auch extrakorporale Stoßwellendiagnostik (ESWD) genannt.

In einer vorteilhaften Ausführungsform des Verfahrens wird einer der Werte etwa eine Stunde nach der Bestrahlung ermittelt. Mit anderen Worten liegen erster oder zweiter Zeitpunkt etwa eine Stunde nach der Bestrahlung der Körperregion mit akustischer Energie. Erfahrungsgemäß steigt der Kennwert, z.B. PSA etwa eine Stunde nach der Irritation der Köperregion auf ein Maximum an, so dass in diesem Fall ein Maximalwert für den Kennwert ermittelbar ist. Das erfindungsgemäße Verfahren ist daher für den ambulanten Betrieb geeignet.

Liegt der erste Zeitpunkt etwa eine Stunde nach der Bestrahlung, dann wird der zweite Zeitpunkt z.B. so spät gewählt, dass schon wieder ein Abfall des zweiten zum ersten Wertes zu beobachten ist.

Ist es der zweite Zeitpunkt, so bietet es sich an, den ersten Zeitpunkt so zu legen, dass dort ein Referenzwert als Basiswert ermittelt wird. In einer vorteilhaften Ausführungsform wird daher der erste Wert vor der Bestrahlung ermittelt. Der erste Zeitpunkt liegt damit also vor der Bestrahlung und der erste Wert dient als Referenzwert für den erfindungsgemäß nach der Bestrahlung gemessenen zweiten Wert. So kann beispielsweise die maximale Erhöhung des Kennwertes zu einem Basiswert ermittelt werden.

Für die Bestimmung der Tumorkenngröße aus den Werten existieren vielfältige Möglichkeiten, von denen im Folgenden vorteilhafte Beispiele genannt sein sollen:
In einer vorteilhaften Ausführungsform des Verfahrens wird die Tumorkenngröße als Quotient aus zweitem und erstem Wert ermittelt. Die Tumorkenngröße besagt dann z.B., auf das wievielfache seines Ausgangswertes bzw. Referenzwertes der Kennwert durch die Irritation erhöht wurde.
In einer anderen Ausführungsform des Verfahrens werden neben erstem und zweitem Wert noch weitere, also mehrere Messwerte zu verschiedenen Zeitpunkten ermittelt und die Tumorkenngröße als zeitlicher Verlauf der Werte ermittelt. Liegen die Zeitpunkte nahe genug beieinander, kann z.B. der zeitliche Verlauf von Erhöhung bzw. Abfall des Kennwertes vor, während oder nach der mechanischen Irritation grafisch dargestellt werden. Die Tumorkenngröße ist dann beispielsweise wieder die maximale Überhöhung der Kurve über einem Basiswert, die Steigung der Kurve in bestimmten Punkten, Anstiegs- oder Abfallzeiten des Kennwerts auf bestimmte Prozentwerte des Maximums.
In einer bevorzugten Ausführungsform des Verfahrens wird als Kennwert ein Tumormarker im Blutserum, z.B. PSA, AFP oder bHCG ermittelt, wie oben erläutert.

Wie ebenfalls oben erläutert, wird in einer weiteren Ausführungsform des Verfahrens die Tumorkenngröße für die Prostata, die Hoden oder die Mamma als Körperregion ermittelt.

In einer weiteren Ausführungsform des Verfahrens wird die Körperregion mit akustischer Energie in Form von diagnostischem oder fokussiertem Ultraschall, Stoß- oder Druckwellen bestrahlt. Die genannten Verfahren eignen sich besonders gut zur Einbringung mechanischer Energie in bestimmte Körperregionen des Patienten, die zu untersuchen sind. Durch das erfindungsgemäße Verfahren ergibt sich neben der bisher bekannten Verwendung in der Therapie eine neue Anwendungsmöglichkeit für bereits bestehende Geräte wie Lithotriptoren, also Stoßwellen-Applikatoren. Dies führt zu einer Mehrnutzung der Geräte und damit zu einem Kostenvorteil.

In einer weiteren Variante des Verfahrens werden Microbubbles in der Körperregion oder in unmittelbarer Nähe der Körperregion zerstört, wodurch die Körperregion mit akustischer Energie bestrahlt wird. Diese Verfahrensvariante nutzt in kontrollierter Weise einen bei sonstigen Ultraschalltherapien unerwünschten Effekt, der z.B. zu Gewebeschäden am Patienten führen kann.

Die Microbubbles werden hierbei z.B. durch ein systemisch verabreichtes US-Kontrastmittel erzeugt und dann mit Hilfe von Ultraschall kontrolliert zerstört.

In einer weiteren Ausführungsform des Verfahrens wird die Körperregion mit akustischer Energie aus einer perkutan- oder transrektal am Patienten platzierten Quelle bestrahlt. Beide Verfahren sind nichtinvasiv und eignen sich z.B. besonders in Verbindung mit der Anwendung des Verfahrens auf die Prostata.

Hinsichtlich des Medizingeräts wird die Aufgabe der Erfindung gelöst durch ein Medizingerät gemäß Patentanspruch 13, welches zur Ausführung des oben genannten erfindungsgemäßen Verfahrens geeignet ist. Das Medizingerät wurde damit im wesentlichen bereits im Zusammenhang mit dem erfindungsgemäßen Verfahren erläutert. Die im folgenden genannten Begriffe beziehen sich daher auf das oben Gesagte. Das Medizingerät weist eine Quelle für akustische Energie auf und enthält eine Auswerteeinheit mit einem Eingang, über welchen die Auswerteeinheit die zum ersten und zweiten Zeitpunkt ermittelten Werte des Kennwertes des Patienten empfängt. Die Auswerteeinheit ist außerdem zur Ermittlung der Tumorkenngröße aus den Änderungen zwischen erstem und zweitem Wert ausgebildet.

In einer bevorzugten Ausführungsform weist das Medizingerät ein Bildgebungsgerät auf, welches zur Ortung der Körperregion und zur Ausrichtung der Quelle auf die Körperregion dient. Das Medizingerät kann damit ohne Unterstützung durch zusätzliche Geräte betrieben werden und dennoch exakt die beabsichtigte Körperregion finden und an dieser das erfindungsgemäße Verfahren durchführen.

In einer besonders bevorzugten Ausführungsform ist das Bildgebungsgerät ein Ultraschallbildgebungsgerät, welches für den Patienten hinsichtlich Strahlenbelastung unbedenklich ist.

In einer weiteren Ausführungsform umfasst das Medizingerät außerdem eine In-Vitro-Testeinheit zur Ermittlung des Wertes des Kennwertes des Patienten. Der Kennwert bzw. dessen Wert kann damit direkt im Medizingerät ermittelt werden. Besonders für Kennwerte aus Körperflüssigkeiten müssen diese, z.B. Urin- oder Blutproben des Patienten, nicht zuerst in ein Labor zur Auswertung gesendet werden, um die ermittelten Werte anschließend im erfindungsgemäßen Verfahren weiter verwenden zu können.

Ein erstes Anwendungsgebiet für das erfindungsgemäße Verfahren ist die Vorsorge bzw. Erstdiagnostik als Provokationstest. Zum ersten Zeitpunkt - vor der Energieapplikation in die Körperregion - wird die sogenannte "Tumormarker baseline" gemessen. Nach der Irritation - z.B. der Prostata - wird der postapplikative Wert des Tumormarkers und der Quotient beider Werte bestimmt. Ein überdurchschnittlicher Anstieg des Wertes, also ein hoher Quotient, könnte hierbei z.B. den Arzt im Rahmen seiner Diagnose auf ein Karzinom oder zumindest auf ein erhöhtes Risiko eines solchen hinweisen. Alternativ kann auch der zeitliche Verlauf des Tumormarkers gemessen werden. Es ist auch denkbar, die Irritation durch wiederholte Energieapplikation - ggf. mit steigender akustischer Energie - öfters durchzuführen. Auch kann alternativ das Abklingen der vorübergehenden Erhöhung des Tumormarkers als Tumorkenngröße ermittelt werden.

Ein weiteres Anwendungsgebiet ist die substitutive Diagnostik bei operativen Eingriffen. Anstelle einer operativen Lymphknotenentfernung bei Tumoren erfolgt dann eine Applikation von akustischer Energie - z.B. auf die Lymphknoten im Becken. Im Falle eines PSA-exprimierenden Tumors ist es durchaus denkbar, dass durch Bewertung der mit dem erfindungsgemäßen Verfahren erzeugten Tumorkenngröße im Rahmen einer Diagnose eine Metastasierung ausgeschlossen oder nachgewiesen werden könnte. So stützt eine signifikante Erhöhung des PSA-Wertes nach einer Irritation der Lymphabflussbahnen bzw. iliakalen Lymphknoten mit akustischer Energie die Diagnose eines Arztes bei einem eindeutigen und nicht invasiven Nachweis von Lymphknotenmetastasen eines Prostatakarzinoms.

Für eine weitere Beschreibung der Erfindung wird auf die Ausführungsbeispiele der Zeichnungen verwiesen. Es zeigt in einer schematischen Prinzipskizze:

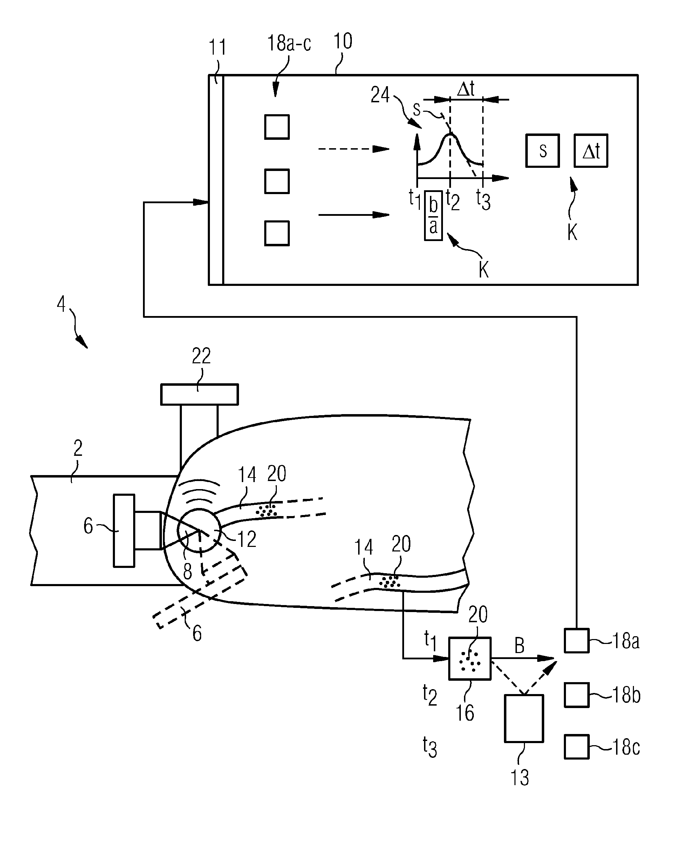

Fig. 1 ein Medizingerät beim Einsatz an einem Patienten bei der Ausführung des erfindungsgemäßen Verfahrens.

Fig. 1 zeigt einen Patienten 2, an dem eine ESWD durchgeführt wird. Das hierzu verwendete Medizingerät 4 umfasst eine Quelle 6 für akustische Energie 8, dargestellt durch einen Strahlkegel, sowie eine Auswerteeinheit 10. Im Beispiel möchte ein Arzt eine Tumordiagnose für die Körperregion 12, im Beispiel die Prostata des Patienten 2 erstellen und benutzt das erfindungsgemäße Verfahren, um eine Tumorkenngröße zu ermitteln, auf die er dann seine Diagnose stützen kann.

Vor Beginn der ESWD wird dem Patienten eine Körperflüssigkeit, im Beispiel aus dessen Blutkreislauf 14 zu einem Zeitpunkt t1 Blut 16 entnommen und hieraus ein erster Wert 18a eines Kennwertes B, im Beispiel als Blutkennwert der Wert der Konzentration von PSA 20 zu 0,41 ng/ml, ermittelt.

Anschließend wird die Körperregion 12 durch die perineal angesetzte Quelle 6 mit akustischer Energie 8 beschossen, woraufhin diese mehr PSA 20 in den Blutkreislauf 14 ausschüttet. Der Beschuss wird mit einer Energie 8 von 5,3J in 766 Einzelschüssen mit einer Stoßwellenstärke von 0,1 bis 1 durchgeführt. Vom gesamten Volumen der Körperregion 12, im Falle der Prostata ca. 30ml, werden hierbei lediglich zwei Foki, also Zielpunkte bzw. Zielgebiete in je einem Prostatalappen beschossen.

Um die Körperregion 12 mit Energie 8 gezielt zu erreichen, weist das Medizingerät 4 ein Bildgebungsgerät 22 auf. Mit Hilfe dessen Hilfe wird während des Beschusses eine Ortung der Körperregion 12 durchgeführt. Es wird eine Kontrastmitteldarstellung der prostatischen Harnröhre und eine Durchleuchtung nach Art eines Cysturethrogramms (CUG) durchgeführt.

Zum Zeitpunkt t2 wird erneut eine zweiter Wert 18b des Kennwertes B, also der Konzentration von PSA 20 im Blut 16 zu 0,47 ng/ml, ermittelt. Durch den Beschuss wurde also der PSA-Wert um 15% erhöht. Der Zeitpunkt t2 ist hierbei etwa eine Stunde nach Einbringung der Energie 8 gelegt, um einen maximal erhöhten Wert von PSA festzustellen.

Die Werte 18a, b werden in einer ersten Ausführungsform in einem Labor ermittelt. Die vom Labor rückgelieferten Werte werden dann an einem Eingang 11 der Auswerteeinheit 10 zugeführt. Die Tumorkenngröße K wird dann als Quotient der Werte 18b zu 18a K=0,47/0,41=1,146 ermittelt. Dieser Wert dient dem Arzt nun als Grundlage für seine Diagnose, ob ein Prostatakarzinom vorliegt.

In einer alternativen Ausführungsform enthält das Medizinsystem 4 eine In-Vitro-Testeinheit 13, die die Werte 18a-c aus dem Blut 16 ermittelt.

Nach der ESWD ist am Patienten eine deutliche Makrohämaturie und Dysurie festgestellt. Dies ist ein weiteres Anzeichen dafür, dass die Stoßwellen aus der Quelle 6 die Prostata 12 sicher erreicht haben.

In einer alternativen Ausführungsform wird die Körperregion 12 flächendeckend bestrahlt bzw. durch die Energie 8 erschüttert. Dabei wird entsprechend mehr PSA 20 freigesetzt und der Wert 18b steigt im Verhältnis zum Wert 18a noch stärker an.

Bei der in Fig. 1 gezeigten perinealen ESWL, also dem Ansetzen der Quelle 6 am Perineum der Patienten 2, ist es aufgrund des Knochenfensters in Form der Sitzbeinhöcker allerdings schwierig, die Prostata besonders im rektumnahen Bereich zu beschießen. Knochenschmerzen, Analschmerzen und Beckenbodenkrämpfe sind herbei als Nebenwirkungen zu beobachten. Das Perineum ist daher als Zugangsweg für die ESWL unter Umständen nicht geeignet.

Fig. 1 zeigt daher eine alternative Quelle 6, welche dem Patienten 2 transrektal eingeführt ist, um die Energie 8 auf anderem Weg zur Körperregion 12 auszusenden.

In einer Ausführungsform ist die Quelle 6 ein endorektaler transrektaler Ultraschall (TRUS)-Scanner, der bei einer Frequenz von 7,5 MHz arbeitet. Der Scanner ist extern durch einen in der Rima ani laufenden Halter fixiert und ist mit einem Ballon überzogen. Ein solcher flüssigkeitsgefüllter Ballon mit einem Volumen von z.B. unter 150cm³ kann volumenvariiert werden.

In einer alternativen Ausführungsform dient dieser TRUS-Scanner auch als Bildgebungsgerät: Der Scanner erstellt in transversalen Schnitten durch einen cranio-caudalen Scanprozess Schnittbilder. Bei einer Dauer von etwa 15s errechnet er dann ein Organmodell und bildet es auf einem nicht dargestellten Bildschirm ab. Die Quelle 6 kann so gleichzeitig zur Bildgebung benutzt werden.

Somit ist es auch möglich, einen bekannten TRUS-Scanner zur Reizdiagnostik bzw. zu einer Doppelfunktion umzurüsten: Hierzu muss lediglich der Scanner durch einen Applikator ersetzt werden, welcher einen entsprechend höheren Schalldruck erzeugen kann, z.B. einen ARFI-Applikator (Acoustic Radiation Force Imaging). Mit einem konventionellen TRUS-Scanner ausgestattet, kann die Quelle 6 dann gleichzeitig als intraoperatives Navigationssystem funktionieren.

Die benötige Reizenergie in Form der Energie 8 ist hierbei etwa gleich derer beim perinealen Zugang Aufgrund der örtlichen Nähe muss aber gegebenenfalls eine geringere Energie appliziert werden, da weniger Absorption und Streuung auftritt. Im Beispiel hat sich gezeigt, dass die Energieeinkopplung in die Körperregion 12 fächerförmig sein sollte, z.B. mit einer Fächerbreite von 2cm. Die Energieeinkopplung sollte hierbei nicht fokussiert sein und die wirksame Eindringtiefe sollte maximal 5cm betragen.

Zu einem weiteren Zeitpunkt t3, 24 Stunden nach der Applikation der Energie 8, wird ein weiterer Wert 18c im Blut 16 des Patienten bestimmt, welcher wieder dem Ausgangswert 18a entspricht, d.h. der künstlich erhöhte Wert von PSA 20 ist dann nicht mehr nachweisbar.

In verschiedenen Ausführungsformen enthält das Bildgebungsgerät also zwei Transducer oder einen einzigen, der dann mit verschiedenen Energien für Bildgebung und Bestrahlung betreibbar ist.

In einer alternativen Ausführungsform werden die zu den Zeitpunkten t1-t3 aufgenommenen Werte 18a-c und eine Vielzahl von Zwischenwerten des Kennwertes B als zeitlicher Verlauf 24 in Form eines Diagramms über der Zeit dargestellt. Die Tumorkenngröße K wird dann ebenfalls als Änderung zwischen den einzelnen ermittelten Werten des Kennwertes B ermittelt. In diesem Fall z.B. als Steigung s der abfallenden Kurve des Blutkennwertes nach dem Zeitpunkt t2 oder als Zeitintervall Δt als Zeit zwischen dem Maximum des Verlaufs 24 bis der Kennwert B wieder auf den ursprünglich zum Zeitpunkt t1 gemessenen Wert abgefallen ist.

## Patentansprüche

1. Verfahren zur Ermittlung einer auf eine Körperregion (12) eines Patienten (2) bezogenen Tumorkenngröße (K), bei dem:
- die Körperregion (12) zum Zweck einer mechanischen Irritation mit akustischer Energie (8) bestrahlt wird,
- zu einem ersten Zeitpunkt (t1) ein erster Wert (18a) eines mit der Irritation korrelierten Kennwertes (B) des Patienten (2) ermittelt wird,
- zu einem nach dem ersten Zeitpunkt (t1) und nach der Bestrahlung liegenden zweiten Zeitpunkt (t2) ein zweiter Wert (18b) des Kennwertes (B) ermittelt wird,
- die Tumorkenngröße (K) aus der Änderung zwischen erstem (18a) und zweitem Wert (18b) ermittelt wird.

2. Verfahren nach Anspruch 1, bei dem als Kennwert (B) ein solcher gewählt wird, der aus einer Körperflüssigkeit (16) des Patienten (2) bestimmbar ist.

3. Verfahren nach Anspruch 1 oder 2, bei dem einer der Werte (18a,b) zum ersten (t1) oder zweiten Zeitpunkt (t2) etwa eine Stunde nach der Bestrahlung des Patienten (2) mit akustischer Energie (8) ermittelt wird.

4. Verfahren nach einem der vorhergehenden Ansprüche, bei dem der erste Wert (18a) vor der Bestrahlung des Patienten (2) mit akustischer Energie (8) ermittelt wird.

5. Verfahren nach einem der vorhergehenden Ansprüche, bei dem die Tumorkenngröße (K) als Quotient aus zweitem (18b) und erstem Wert (18a) ermittelt wird.

6. Verfahren nach einem der vorhergehenden Ansprüche, bei dem mehrere Werte (18a-c) des Kennwertes (B) zu verschiedenen Zeitpunkten (t1-3) ermittelt werden und die Tumorkenngröße (K) als oder anhand des zeitlichen Verlaufs (24) der Werte (18a-c) ermittelt wird.

7. Verfahren nach einem der vorhergehenden Ansprüche, bei dem als Kennwert (B) ein Tumormarker im Blutserum des Patienten (2) ermittelt wird.

8. Verfahren nach Anspruch 7, bei dem als Tumormarker PSA, AFP oder bHCG ermittelt wird.

9. Verfahren nach einem der vorhergehenden Ansprüche, bei dem die Tumorkenngröße (K) für Prostata, Hoden oder Mamma als Körperregion (12) des Patienten (2) ermittelt wird.

10. Verfahren nach einem der vorhergehenden Ansprüche, bei dem die Körperregion (12) mit akustischer Energie (8) in Form von diagnostischem oder fokussiertem Ultraschall, oder Stoß- oder Druckwellen bestrahlt wird.

11. Verfahren nach einem der vorhergehenden Ansprüche, bei dem die Körperregion (12) durch Zerstören von Microbubbles in der oder in unmittelbarer Nähe der Körperregion (12) mit akustischer Energie (8) bestrahlt wird.

12. Verfahren nach einem der vorhergehenden Ansprüche, bei dem die Körperregion (12) mit akustischer Energie (8) aus einer perkutan oder transrektal am Patienten (2) platzierten Quelle (6) bestrahlt wird.

13. Medizingerät (4) zur Ermittlung einer auf eine Körperregion (12) eines Patienten (2) bezogenen Tumorkenngröße (K), mit
- einer Quelle (6) für akustische Energie (8), die zum Zweck einer mechanischen Irritation auf die Körperregion (12) abstrahlbar ist,
- einer Auswerteeinheit (10) mit einem Eingang (11) für einen zu einem ersten Zeitpunkt (t1) ermittelten ersten Wert (18a) eines mit der Irritation korrelierten Kennwertes (B) des Patienten (2), und für einen zu einem nach dem ersten Zeitpunkt (t1) und nach der Bestrahlung liegenden zweiten Zeitpunkt (t2) ermittelten zweiten Wert (18b) des Kennwertes (B), wobei die Auswerteeinheit (10) zur Ermittlung der Tumorkenngröße (K) aus der Änderung zwischen erstem (18a) und zweitem Wert (18b) dient.

14. Medizingerät (4) nach Anspruch 13, mit einem Bildgebungsgerät (22) zur Ortung der Körperregion (12) und zur Ausrichtung der Quelle (6) auf die Körperregion (12).

15. Medizingerät (4) nach Anspruch 13 oder 14, bei dem das Bildgebungsgerät (22) ein Ultraschallbildgebungsgerät ist.

16. Medizingerät (4) nach einem der Ansprüche 13 bis 15, mit einer In-Vitro-Testeinheit (13) zur Ermittlung des Wertes (18a-c) des Kennwertes (B) des Patienten (2).
